# EUROPEAN PATENT SPECIFICATION

(11) **EP 1 195 159 B1**
(45) Date of publication and mention of the grant of the patent: **31.05.2006**
(21) Application number: 00121950.0
(22) Date of filing: 09.10.2000
(51) Int. Cl.: A61K 31/195, A61K 31/375, A61P 35/00

(54) **Therapeutic combination of ascorbate with lysine and arginine for prevention and treatment of cancer**
Therapeutische Kombination des Ascorbats mit Lysin und Arginin für Verhinderung und Behandlung des Krebses
Combinaison thérapeutique d'ascorbate avec de la lysine et de l'arginine pour la prévention et le traitement du cancer

(43) Date of publication of application: 10.04.2002
(73) Proprietor: Rath, Matthias, Dr. med., 7609 KL Almelo (NL)
(72) Inventor: Rath, Matthias, Dr. med., 7609 KL Almelo (NL)
(74) Representative: Federhen, Ludwig

(56) References cited:
- EP-A- 0 891 771
- DE-A- 3 440 090
- GB-A- 2 029 220
- GB-A- 2 268 871
- US-A- 5 198 465
- US-A- 5 626 883
- US-A- 5 650 418
- US-A- 5 891 459
- KATZ E.A.: "Reduction of cholesterol and Lp(a) and regression of coronary artery disease: A case study." JOURNAL OF ORTHOMOLECULAR MEDICINE, (1996) 11/3 (173-179)., XP000972356
- BOSTOM A. G.: "The effect of high dose ascorbate supplementation on plasma lipoprotein (a) levels in patients with premature coronary heart disease" PHARMACOTHERAPY, vol. 15, no. 4, July 1995 (1995-07), pages 458-464, XP000972298

## Description

The cause of many forms of cancer remains unknown. However, irrespective of their causative agent, organ of origin or histological type, all forms of cancer spread with the help of the tissue-dissolving mechanism. Cancer cell enzymes produce and secrete enzymes that destroy the surrounding connective tissue that would otherwise keep the cancer cells confined.

Research has established that the more enzymes a cancer cell produces, the more aggressively the cancer develops. The faster the cancer can spread through a body, the shorter the life expectancy of the patient if the mechanism is not stopped.

The collagen-dissolving mechanism also plays a decisive role in the spread of cancer and the growth of secondary tumors in other organs or parts of the body (metastasis). The more collagen-digesting enzymes (collagenases) a specific cancer cell can activate, the faster secondary tumors will develop and the worse the shorter the remaining life-span for the patient.

The most important substances able to block the cascade that leads to collagenase digestion of the connective tissue is the natural amino acid L-lysine. Lysine prevents these enzymes from uncontrollably disintegrating connective tissue. This way the spread of diseases can be slowed down or stopped.

In addition to this mechanism, progress has been made in understanding the metabolism of cells and the role of certain biochemical compounds in maintaining their proper function. Consequently, correcting the dysfunction of cellular metabolism would be a key to reducing susceptibility to cancer in different organs. I further assumed that the cellular dysfunction could be caused by a deficiency of certain biochemical compounds needed as coenzymes in the Krebs-cycle, the respiration chain and for other metabolic functions in smooth muscle cells.

EP 0 891 771 discloses pharmaceutical compositions comprising lysine or lysine salts and ascorbate compounds which are used, among others, for the treatment of cancer metastasis. DE 34 40 090 discloses a composition for cancer treatment consisting of three different solutions (A, B and C). Solution A comprises cysteine and methionine, solution B comprises histidin, phenylalanine and lysine, and solution C comprises tryptophan, valine, leucine, threonine and ascorbic acid. By stabilizing the pH-value of these solutions to a certain value, an inhibition of growth and even degeneration of malignant tumors is achieved. GB 2 029 220 discloses amino acid solutions comprising essential amino acids like lysine and non-essential amino acids like arginine as well as ascorbic acid in a vitamin mixture for patients with cancer. US 5,626,883 describes a vitamin supplement comprising ascorbic acid, ascorbyl palmitate, niacinamid ascorbate, calcium ascorbate, magnesium ascorbate, potassium ascorbate, and sodium ascorbate which together can be administered to a human to avoid the transitory initial suppression of human NK-cell activity. GB 2 268 871 describes a composition for use as a food or food supplement comprising a proteinaceous component and a nutrient component selected from a vitamin, minetal, trace element or mixture thereof. US 5,891,459 discloses a dietary supplement comprising arginine or arginine hydrochloride or lysine or a mixture of the two and an antioxidant, for example vitamin C, vitamin E or carotene, to enhance the level of endogenous nitric oxide or other intermediates in the nitric oxide induced relaxation pathway in the host. None of these documents describe the particular ingredients and the particular amounts of the composition according to the present invention.

The concept according to the present invention was successfully tested for the first forms of cancer. Following is a first case report documenting the efficacy of this therapeutic approach in the treatment of esophagus cancer.

In August 1999 the patient was diagnosed with a tumor in and around the esophagus. In September the patient was admitted to hospital but surgery showed that the tumor could not be removed as it had spread around the trachea and the aorta. The doctors decided on chemotherapy, 33 cobalt radiation treatments.

In two weeks the patient lost a stone in weight and could not leave her bed.

After the treatments it was found that the esophagus had been damaged, there was a long rip towards the stomach. Early December 1999 the patient was given an artificial stomach opening. She was sent home with the remark that she probably would not survive past Christmas.

From that moment on the patient started the treatment with the composition of compounds listed in Table 1.

On January 10^{th} she returned to hospital for a medical exam. It was established that the tumor had stopped growing, that the patient had gained some weight and that she could walk again. In the meantime she is able to do some housework.

The last exam in March 2000 showed that the esophagus lesions are decreasing. Must significantly, the lung metastases disappeared and the fistulas in the esophagus are diminishing. Meanwhile, three doctors have told the patient that a complete remission and covery is now a distinct possibility.

Therefore the claim of this patent is that a preventive and therapeutic composition has been identified that is able to prevent and treat esophagus cancer.

**Table 1 :**

| Vitamin C as: | | |
|---|---|---|
| Ascorbic Acid | mg | 2180 |
| Ascorbyl Palmitate | mg | 620 |
| Calcium Ascorbate | mg | 2000 |
| Magnesium Ascorbate | mg | 2000 |
| Biotin | mcg | 65 |
| Vitamin B1 (ThiamineHCl) | mg | 7 |
| Vitamin B2 (Riboflavin) | mg | 7 |
| Vitamin B3 (Niacin) | mg | 10 |
| Vitamin B3 (Niacinamid) | mg | 35 |
| Vitamin B5 (d-Calcium Pantothenate) | mg | 40 |
| Vitamin B6 (PyridoxineHCl) | mg | 18 |
| Vitamin B12 (Cyanocobalamin) | mcg | 50 |
| Vitamin D3 (Cholecalciferol) | I.U. | 130 |
| Vitamin A (Beta Carotene) | I.U. | 4165 |
| Vitamin E (d-Alpha Tocopherol) | I.U. | 330 |
| Folic Acid | mcg | 490 |
| L-Proline | mg | 560 |
| L-Lysine (HCl) | mg | 4010 |
| L-Carnitine | mg | 35 |
| L-Arginine (HCl) | mg | 790 |
| L-Cysteine (HCl Monohydrate) | mg | 35 |
| Calcium (Glycinate/Citrate) | mg | 535 |
| Magnesium (Glycinate/Citarte) | mg | 290 |
| Potassium | mg | 20 |
| Zinc (Glycinate) | mg | 7 |
| Manganese (Chelate) | mg | 1.3 |
| Copper (Glycinate) | mcg | 330 |
| Selenium (L-Selanomethionine) | mcg | 20 |
| Chromium (Glycinate) | mcg | 10 |
| Molybdenum (Glycinate) | mcg | 4 |
| Inositol | mg | 35 |
| Coenzyme Q10 | mg | 7 |
| Phosphorus (Dicalcium Phosphate Anhydrous) | mg | 15 |
| Pycnogenol | mg | 7 |
| Citrus Bioflavonoids | mg | 1150 |
| Beta-, Gamma-, Delta-Tocophenol-Mix Carotinoid-Mix: | mg | 22 |
| (Alpha-Carotene, Lutein, Zea-Kryptoxanthin) | mcg | 125 |
| Iron (Fumarate) | mg | 10 |

## Claims

1. Composition of biochemical substances comprising
| | |
|---|---|
| ascorbic acid | 2180 mg |
| ascorbyl palmitate | 620 mg |
| calcium ascorbate | 2000 mg |
| magnesium ascorbate | 2000 mg |
| biotin | 65 mcg |
| vitamin B1 (thiamineHCl) | 7 mg |
| vitamin B2 (riboflavin) | 7 mg |
| vitamin B3 (niacin) | 10 mg |
| vitamin B3 (niacinamid) | 35 mg |
| vitamin B5 (d-calcium pantothenate) | 40 mg |
| vitamin B6 (pyridoxineHCl) | 18 mg |
| vitamin B12 (cyanocobalamin) | 50 mcg |
| vitamin D3 (cholecalciferol) | 130 I.U. |
| vitamin A (beta carotene) | 4165 I.U. |
| vitamin E (d-alpha tocopherol) | 330 I.U. |
| folic acid | 490 mcg |
| L-proline | 560 mg |
| L-lysine (HCl) | 4010 mg |
| L-carnitine | 35 mg |
| L-arginine (HCl) | 790 mg |
| L-cysteine (HCl monohydrate) | 35 mg |
| calcium (glycinate/citrate) | 535 mg |
| magnesium (glycinate/citrate) | 290 mg |
| potassium | 20 mg |
| zinc (glycinate) | 7 mg |
| manganese (chelate) | 1.3 mg |
| copper (glycinate) | 330 mcg |
| selenium (L-selenomethionine) | 20 mcg |
| chromium (glycinate) | 10 mcg |
| molybdenum (glycinate) | 4 mcg |
| inositol | 35 mg |
| coenzyme Q10 | 7 mg |
| phosphorus (dicalcium phosphate anhydrous) | 15 mg |
| pycnogenol | 7 mg |
| citrus bioflavonoids | 1150 mg |
| beta-, gamma-, delta-tocopherol-mix | 22 mg |
| carotinoid-mix: (alpha-carotene, lutein, zea-kryptoxanthin) | 125 mcg |
| iron (fumarate) | 10 mg |

2. Use of a composition according to claim 1 for the preparation of a pharmaceutical composition for prevention or treatment of esophagus cancer.

## Patentansprüche

1. Zusammensetzung biochemischer Substanzen, die das folgende umfasst:
| | |
|---|---|
| Ascorbinsäure | 2180 mg |
| Ascorbylpalmitat | 620 mg |
| Calciumascorbat | 2000 mg |
| Magnesiumascorbat | 2000 mg |
| Biotin | 65 mcg |
| Vitamin B (Thiamin-HCl) | 7 mg |
| Vitamin B2 (Riboflavin) | 7 mg |
| Vitamin B3 (Niacin) | 10 mg |
| Vitamin B3 (Niacinamid) | 35 mg |
| Vitamin B5 (d-Calciumpantothenat) | 40 mg |
| Vitamin B6 (Pyridoxin-HCl) | 18 mg |
| Vitamin B12 (Cyanocobalamin) | 50 mcg |
| Vitamin D3 (Cholecalciferol) | 130 I.U. |
| Vitamin A (Betacarotin) | 4165 I.U. |
| Vitamin E (d-alpha-Tocopherol) | 330 I.U. |
| Folsäure | 490 mcg |
| L-Proline | 560 mg |
| L-Lysine (HCl) | 4010 mg |
| L-Carnitin | 35 mg |
| L-Arginin (HCl) | 790 mg |
| L-Cystein (HCl-Monohydrat) | 35 mg |
| Calcium (Glycinat/Citrat) | 535 mg |
| Magnesium (Glycinat/Citrat) | 290 mg |
| Kalium | 20 mg |
| Zink (Glycinat) | 7 mg |
| Mangan (Chelat) | 1,3 mg |
| Kupfer (Glycinat) | 330 mcg |
| Selen (L-Selenmethionin) | 20 mcg |
| Chrom (Glycinat) | 10 mcg |
| Molybdän (Glycinat) | 4 mcg |
| Inositol | 35 mg |
| Coenzym Q10 | 7 mg |
| Phosphor (Dicalciumphosphat anhydriert) | 15 mg |
| Pycnogenol | 7 mg |
| Zitrusbioflavonoide | 1150 mg |
| beta-, gamma-, delta-Tocopherolgemisch | 22 mg |
| Carotinoidgemisch: (alpha-Carotin, Lutein, Zea-Kryptoxanthin) | 125 mcg |
| Eisen (Fumarat) | 10 mg |

2. Verwendung einer Zusammensetzung gemäß Anspruch 1 für die Herstellung einer pharmazeutischen Zusammensetzung für die Vorbeugung oder Behandlung von Speiseröhrenkrebs.

## Revendications

1. Composition de substances biochimiques comprenant
| | |
|---|---|
| acide ascorbique | 2180 mg |
| palmitate d'ascorbyle | 620 mg |
| ascorbate de calcium | 2000 mg |
| ascorbate de magnésium | 2000 mg |
| biotine | 65 mcg |
| vitamine B1 (thiamineHCl) | 7 mg |
| vitamine B2 (riboflavine) | 7 mg |
| vitamine B3 (niacine) | 10 mg |
| vitamine B3 (niacinamide) | 35 mg |
| vitamine B5 (d-pantothénate de calcium) | 40 mg |
| vitamine B6 (pyridoxineHCl) | 18 mg |
| vitamine B12 (cyanocobalamine) | 50 mcg |
| vitamine D3 (cholécalciférol) | 130 U.I. |
| vitamine A (bêta carotène) | 4165 U.I. |
| vitamine E (d-alpha tocophérol) | 330 U.I. |
| acide folique | 490 mcg |
| L-proline | 560 mg |
| L-lysine (HCl) | 4010 mg |
| L-carnitine | 35 mg |
| L-arginine (HCl) | 790 mg |
| L-cystéine (HCl monohydrate) | 35 mg |
| calcium (glycinate/citrate) | 535 mg |
| magnésium (glycinate/citrate) | 290 mg |
| potassium | 20 mg |
| zinc (glycinate) | 7 mg |
| manganèse (chélate) | 1,3 mg |
| cuivre (glycinate) | 330 mcg |
| sélénium (L-sélénométhionine) | 20 mcg |
| chrome (glycinate) | 10 mcg |
| molybdène (glycinate) | 4 mcg |
| inositol | 35 mg |
| coenzyme Q10 | 7 mg |
| phosphore (phosphate dicalcique anhydre) | 15 mg |
| pycnogénol | 7 mg |
| bioflavonoïdes d'agrumes | 1150 mg |
| bêta-, gamma-, delta-tocophérol en mélange | 22 mg |
| caroténoïdes en mélange (alpha-carotène, lutéine, kryptoxanthine-zéa) | 125 mcg |
| fer (fumarate) | 10 mg |

2. Utilisation d'une composition selon la revendication 1, pour la préparation d'une composition pharmaceutique destinée à la prévention ou au traitement du cancer de l'oesophage.
